(19)

**Europäisches Patentamt**
**European Patent Office**
**Office européen des brevets**

(11) **EP 4 509 526 A2**

(12) **EUROPEAN PATENT APPLICATION**

(43) Date of publication:
**19.02.2025 Bulletin 2025/08**

(21) Application number: **24190919.1**

(22) Date of filing: **25.07.2024**

(51) International Patent Classification (IPC):
*C07K 16/18* (2006.01)    *G01N 33/53* (2006.01)
*A61P 5/16* (2006.01)

(52) Cooperative Patent Classification (CPC):
**C07K 16/18; A61P 5/16; G01N 33/53;**
C07K 2317/92

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.07.2023 CN 202310922507**

(71) Applicants:
• **Shenzhen Mindray Bio-Medical Electronics Co.,
Ltd.
Shenzhen, Guangdong 518057 (CN)**
• **Hytest Ltd.
20520 Turku (FI)**

(72) Inventors:
• **SEMENOV, Alexander G.
20520 Turku (FI)**
• **FEYGINA, Evgeniya E.
20520 Turku (FI)**

• **SOKOLOVA, Elizaveta E.
20520 Turku (FI)**
• **ZHAN, Chengxiong
Shenzhen, 518057 (CN)**
• **LI, Xiaotong
Shenzhen, 518057 (CN)**
• **TANG, Tao
Shenzhen, 518057 (CN)**
• **CHEN, Puguang
Shenzhen, 518057 (CN)**
• **LI, Ke
Shenzhen, 518057 (CN)**

(74) Representative: **KIPA AB
Drottninggatan 11
252 21 Helsingborg (SE)**

Remarks:
The complete document including Reference
Table(s) and the Sequence Listing(s) can be
downloaded from the EPO website

(54) **ANTI-THYROGLOBULIN ANTIBODY, KIT AND USE THEREOF**

(57)    The present application relates to anti-thyroglobulin antibodies, kits and uses thereof. Specifically, the present application provides anti-thyroglobulin specific antibodies, kits containing the antibodies, as well as uses thereof in detecting the presence or level of thyroglobulin and/or thyroglobulin autoantibodies in a sample.

**EP 4 509 526 A2**

**Description**

**Technical Field**

The present application belongs to the field of molecular immunology, and specifically relates to a specific anti-thyroglobulin antibody, a kit comprising the antibody, and uses thereof.

**Background Art**

Thyroglobulin (Tg) is a protein homodimer of 660 kDa with a high number (about 60) of disulfide bonds per monomer and is heavily glycosylated at 17 glycosylation sites. Tg is produced by thyroid gland follicular cells (thyrocytes) and is stored in the follicular colloid of the gland, comprising up to 80% of total protein in the thyroid. Tg functions as a prohormone for the intra-thyroid synthesis of the iodinated tyrosine hormones thyroxine (T4) and triiodothyronine (T3). Nearly 30 of 66 tyrosine residues of Tg are iodinated; the polypeptide chain proteolysis in lysosomes leads to release of T3 and T4 to the cytoplasm of thyrocytes. T3 and T4 are then subsequently secreted to the bloodstream.

Although present in one out of ten healthy individuals, Thyroglobulin antibodies (TgAbs) are usually associated with autoimmune thyroid diseases (AITD): such as Hashimoto's thyroiditis (HT) or Graves' disease (GD). In addition, TgAbs are also found in differentiated thyroid carcinoma (DTC): positive serum TgAb is more than twice as prevalent in DTC patients than in the general population (25% vs. 10%). This makes TgAbs a prognostic marker for DTC development risk assessment.

As Tg and TgAbs are often present simultaneously in patients' sera, both analytes show mutual interference in immunoassays. Tg is widely used as a biomarker for the follow-up of patients with DTC after thyroidectomy: considering that Tg is a thyroid-specific protein, increased Tg concentrations during follow-up are indicative of tumor recurrence or metastasis. In this setting, the presence of Tg antibodies in the sample might cause falsely low Tg values. The common practice is measuring TgAbs in all the samples subjected to Tg assessment.

The epitopes recognized by human antibodies on the large Tg molecule are usually conformational and cannot be determined using synthetic peptides. Serological studies have shown that there are numerous antigenic epitopes on Tg, and at least 40 such epitopes have been suggested on human Tg. It was also found that TgAbs antibodies from patients with AITD have a different binding patterns than natural TgAbs antibodies. The TgAbs specificities in patients with DTC might also differ from those in AITD or healthy individuals. The fact that various TgAbs are present in single individual's bloodstream is also extremely relevant for clinical implications.

Currently, several immunochemical methods are used for TgAbs measurement, implementing different approaches such as indirect immunoassays, double antigen sandwich immunoassays, or competitive immunoassays. In competitive immunoassays, the labeled antibody, specific to Tg, is used as a tracer; TgAbs in the sample compete with the tracer for binding sites on Tg molecule. The method for TgAbs quantification in human sera developed by B.R.A.H.M.S. Diagnostica GmbH (Bergmann, A, US/5,919,632) is based on human TgAbs purified from human sera as a tracer.

The main concern about utilizing the polyclonal antibodies in immunoassay is the lack of characterization, limited amount of material and its irreproducibility especially in case of human-derived preparations. The reproducibility and robustness of an assay also might become a complication. In case of TgAbs measurement the epitope specificity and affinity of antibodies used as a tracer become crucial for the assay performance. Thus, there is a need for development of an immunoassay method for TgAbs assessment based on comprehensively characterized antibodies with specificity and affinity enabling them to compete with all the major TgAbs present in human population.

**Summary**

The present invention describes a monoclonal antibody specific to human Tg, in particular, the binding characteristics (epitope specificity and affinity) of the antibody are comparable to those of the main autoantibodies of TgAbs present in human population, and can compete with TgAbs in samples for binding to Tg, thereby being applied to the measurement of TgAbs in samples.

Antibody, nucleic acid molecule, expression vector, host cell

In one aspect, the present application provides an antibody or antigen-binding fragment thereof that specifically binds to Tg (thyroglobulin), which comprises:

a heavy chain variable region comprising the following three CDRs: CDR-H1 having an amino acid sequence selected from SEQ ID NOs: 1, 7, 13, 19, 25, 31 and variants thereof, CDR-H2 having an amino acid sequence selected from SEQ ID NOs: 2, 8, 14, 20, 26, 32 and variants thereof, and/or CDR-H3 having an amino acid sequence selected from SEQ ID NOs: 3, 9, 15, 21, 27, 33 and variants thereof; and/or,

a light chain variable region comprising the following three CDRs: CDR-L1 having an amino acid sequence selected from SEQ ID NOs: 4, 10, 16, 22, 28, 34 and variants thereof, CDR-L2 having an amino acid sequence selected from SEQ ID NOs: 5, 11, 17, 23, 29, 35 and variants thereof, and/or CDR-L3 having an amino acid sequence selected from SEQ ID NOs: 6, 12, 18, 24, 30, 36 and variants thereof;

wherein each variant contains an amino acid mutation as compared to the amino acid sequence from which it is derived, and the amino acid mutation is a substitution, deletion or addition of one or more amino acids (e.g., the substitution, deletion or addition of 1, 2, 3, 4 or 5 amino acids); preferably, the substitution is a conservative substitution;

preferably, the variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived;

preferably, the three CDRs contained in the heavy chain variable region and/or the three CDRs contained in the light chain variable region are defined by the Kabat numbering system.

In some embodiments, the antibody or antigen-binding fragment thereof comprises:

(1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

(1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

(1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof; or

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof.

In some embodiments, the heavy chain variable region further comprises a heavy chain framework region (FR). In some embodiments, the light chain variable region further comprises a light chain framework region (FR). The framework region is highly conserved and provides a scaffold for the six CDRs in three-dimensional space to form an antigen binding surface. The variable domains of naturally occurring heavy and light chains each contain four FR regions (FR1, FR2, FR3, and FR4), which primarily use a β-folded configuration and are connected by three hypervariable regions that form loops connecting the β-folded structure and in some cases form a portion of the β-folded structure. The hypervariable regions in each chain are closely together through the FRs and contribute to the formation of the antigen-binding side ends together with the hypervariable regions from another chain (see, Kabat et al., loc. cit.). The heavy chain framework region and/or the light chain framework region can each independently be derived from the framework region of any species immunoglobulin. In some embodiments, the heavy chain framework region and the light chain framework region are each independently derived from the heavy chain framework region and the light chain framework region of a human or mouse immunoglobulin. In some embodiments, the heavy chain framework region and the light chain framework region each independently comprise amino acid sequences derived from the heavy chain framework region and the light chain framework region of a mouse immunoglobulin, the heavy chain framework region and the light chain framework region of a human immunoglobulin, or a combination thereof. In some embodiments, the heavy chain framework region and the light chain framework region each independently comprise amino acid sequences derived from the heavy chain framework region and the light chain framework region of a human germline antibody.

In some embodiments, the antibody or antigen-binding fragment thereof further comprises a heavy chain constant region (CH) and a light chain constant region (CL). In some embodiments, the antibody or antigen-binding fragment thereof comprises a mouse heavy chain constant region and a mouse light chain constant region. In some embodiments, the antibody or antigen-binding fragment thereof comprises a rat heavy chain constant region and a rat light chain constant region. In some embodiments, the antibody or antigen-binding fragment thereof comprises a human heavy chain constant region and a human light chain constant region. In some embodiments, the antibody or antigen-binding fragment thereof is an IgG, IgM, IgE, IgD or IgA antibody. In some embodiments, the heavy chain constant region is an IgG heavy chain constant region, such as an IgG1, IgG2, IgG3 or IgG4 heavy chain constant region, and for example, a mouse IgG1, IgG2a, IgG2b or IgG3 constant region. In some embodiments, the light chain constant region is a κ or λ light chain constant region (e.g., a human λ light chain constant region).

In some embodiments, the antigen-binding fragment is selected from the group consisting of scFv, Fab, Fab', (Fab')$_2$, Fd, Fv, CDR fragment, nanobody, disulfide-bond linked Fv (dsFv), diabody, bispecific antibody and multispecific antibody; and/or, the antibody is a mouse antibody, a chimeric antibody or a humanized antibody.

In some embodiments, the antibody or antigen-binding fragment thereof further has a detectable label.

In the present application, the detectable label can be any substance that can be detected by fluorescent, spectroscopic, photochemical, biochemical, immunological, electrical, optical or chemical means. It is particularly preferred that such labels are suitable for immunological detection (e.g., enzyme-linked immunosorbent assay, radioimmunoassay, fluorescent immunoassay, chemiluminescent immunoassay, etc.). Such labels are well known in the art and include, but are not limited to, enzymes (e.g., oxidase, microperoxidase, horseradish peroxidase, alkaline phosphatase, β-galactosidase, urease, glucose oxidase, etc.), radionuclides (e.g., $^3$H, $^{125}$I, $^{35}$S, $^{14}$C or $^{32}$P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750), europium and green fluorescent protein, etc.), chemiluminescence (e.g., luminol, isoluminol, phenanthridinium, acridinium esters, etc.), and biotin for binding to avidin modified with the above-mentioned labels (e.g., streptavidin). The labels covered in the present application can be detected by methods known in the art. For example, radioactive labels can be detected using photographic film or a scintillation counter, and fluorescent labels can be detected using photodetectors to detect the emitted light. Enzyme labels are generally detected by providing a substrate to the enzyme and detecting the reaction product produced by the action of the enzyme on the substrate. Chemiluminescent substances (e.g., acridinium esters) are generally detected by providing an excitation liquid and/or a catalyst to the luminescent substance to detect the emitted light. Biotin is generally detected by providing biotin with avidin (e.g., streptavidin) modified with the above-mentioned label and detecting the label carried by the avidin linked to biotin. In certain embodiments, the detectable labels as described above can be linked to the antibody or antigen-binding fragment thereof of the present application through linkers of different lengths to reduce potential steric hindrance.

In some embodiments, the detectable label is selected from the group consisting of fluorescein, chemiluminescent substance (e.g., acridinium ester compound), enzyme (e.g., horseradish peroxidase, alkaline phosphatase), radioactive isotope, biotin, colloidal gold, and magnetic particle.

In some embodiments, the antibody or antigen-binding fragment thereof competes with TgAbs (thyroglobulin antibodies) for binding to Tg.

In another aspect, the present application provides an isolated nucleic acid molecule, which encodes the antibody or antigen-binding fragment thereof as described in any of the foregoing.

In another aspect, the present application provides an expression vector, which comprises the isolated nucleic acid molecule as described in any of the foregoing. In some embodiments, the vector is a plasmid, a virus, a phage, a bacterium, or a viroid.

In another aspect, the present application provides a host cell, which comprises the isolated nucleic acid molecule or the expression vector as described in any of the foregoing. In some embodiments, the host cell is an eukaryotic cell, preferably a mammalian cell. In some embodiments, the host cell is a prokaryotic cell, preferably *Escherichia coli.*

In another aspect, the present application provides an antibody or antigen-binding fragment thereof that specifically binds to Tg, wherein the antibody or antigen-binding fragment thereof is:

produced based on a hybridoma cell line deposited with the All-Russian National Collection of Industrial Microorganisms (VKPM) having Accession Number H-223;

produced based on a plasmid in *Escherichia coli* Rosetta™(DE3)pLysS hTG02 VH deposited with VKPM having Accession Number B-14843, and a plasmid in *Escherichia coli* Rosetta™(DE3)pLysS hTG02 VL deposited with VKPM having Accession Number B-14844;

produced based on a plasmid in *Escherichia coli* Rosetta™(DE3)pLysS hTG03 VH deposited with VKPM having Accession Number B-14845, and a plasmid in *Escherichia coli* Rosetta™(DE3)pLysS hTG03 VL deposited with VKPM having Accession Number B-14846;

produced based on a plasmid in *Escherichia coli* Rosetta™(DE3)pLysS hTG04 VH deposited with VKPM having Accession Number B-14847, and a plasmid in *Escherichia coli* Rosetta™(DE3)pLysS hTG04 VL deposited with VKPM having Accession Number B-14848;

produced based on a plasmid in *Escherichia coli* Rosetta™(DE3)pLysS hTG05 VH deposited with VKPM having Accession Number B-14849, and a plasmid in *Escherichia coli* Rosetta™(DE3)pLysS hTG05 VL deposited with VKPM having Accession Number B-14850; or

produced based on a plasmid in *Escherichia coli* Rosetta™(DE3)pLysS hTG06 deposited with VKPM having Accession Number B-14851.

Kit

The consistency between the current mainstream TgAbs detection method and clinical diagnosis needs to be improved, and there are significant differences in the methodological comparisons among different commercial reagents. For patients diagnosed with autoimmune thyroid diseases (Hashimoto's thyroiditis, Graves' disease, etc.), the positive rate of TgAbs varies among different types of diseases, wherein the positive rate of patients diagnosed with Hashimoto's thyroiditis is about 50% to 80%, and the positive rate of patients diagnosed with Graves is about 30% to 50%. Although the detection of Anti-TPO from the same manufacturer can be added to assist the judgment, the positive rate of Anti-TPO varies significantly among different types of patients. Therefore, clinical diagnosis and medication guidance often need to be based on the results of puncture or imaging, but these methods take a long time and cause more pain and burden to patients.

The binding characteristics (epitope specificity and affinity) of the antibody that specifically binds to human Tg in the present application are comparable to those of the main TgAbs autoantibodies in human population, and the antibody can compete with TgAbs in the sample for binding to Tg, and can be used for the measurement of TgAbs. On this basis, the present application provides a kit with a higher clinical consistency rate for in vitro detection of autoimmune thyroid diseases (Hashimoto's thyroiditis, Graves' disease, etc.).

Specifically, one object of the present application is to provide an immunoassay kit, which comprises at least one of the antibody or antigen-binding fragment thereof described above.

One object of the present application is to provide an immunoassay kit, which comprises:

a first reagent, which contains a Tg antigen;
a second reagent, which contains at least one of the antibody or antigen-binding fragment thereof described in any of the foregoing.

In some embodiments, the Tg antigen is a natural or recombinant Tg antigen.

In some embodiments, one of the Tg antigen and the antibody or antigen-binding fragment thereof has a detectable label, and the other is coated on the surface of a solid phase carrier.

In some embodiments, the solid phase carrier is selected from magnetic particles or microtiter plates (e.g., microtiter plates or ELISA plates).

In some embodiments, the detectable label is selected from the group consisting of fluorescein, chemiluminescent label (e.g., acridinium ester compound), enzyme (e.g., horseradish peroxidase, alkaline phosphatase), radioactive isotope, biotin, and colloidal gold.

In some embodiments, the first reagent further contains one or more of the following ingredients: buffer solution, inorganic salt, preservative, surfactant, and stabilizer; and/or

the second reagent further contains one or more of the following ingredients: buffer solution, inorganic salt, preservative, surfactant, and stabilizer.

In some embodiments, the ingredients in the first reagent and the Tg antigen are placed in the same or different formulation units.

In some embodiments, the ingredients in the second reagent and the antibody or antigen-binding fragment thereof are placed in the same or different formulation units.

In some embodiments, the buffer solution is selected from the group consisting of Tris, Hepes, and PBS buffer solution.

In some embodiments, the inorganic salt is one or more selected from the group consisting of NaCl, KCl, $CaCl_2$, $MgCl_2$, and $ZnCl_2$.

In some embodiments, the preservative is selected from the group consisting of Proclin300, PC-300, BND (e.g., BND-10 or BND-99) and BIT (e.g., BIT-10).

In some embodiments, the surfactant is selected from the group consisting of Tween-20, TritonX-100 and S9.

In some embodiments, the stabilizer is selected from the group consisting of calf serum, bovine serum albumin and gelatin.

In some embodiments, the Tg antigen is coated on the surface of the solid phase carrier, and the antibody or antigen-binding fragment thereof has a detectable label.

In some embodiments, the Tg antigen is coated on superparamagnetic microparticles. In some embodiments, the content of the superparamagnetic microparticles in the first reagent is 0.01% to 0.1%, for example, 0.03% to 0.1%, and for example, 0.05% to 0.1%.

In some embodiments, the working concentration of the Tg antigen (referring to the concentration of the Tg antigen in the first reagent when used for measurement) is 0.01% to 0.20%, such as 0.01% to 0.15% or 0.05% to 0.15%.

In some embodiments, the working concentration of the antibody or antigen-binding fragment thereof (referring to the concentration of the antibody or antigen-binding fragment thereof in the second reagent when used for measurement) is 0.05 to 10 $\mu$g/mL, such as 1 to 5 $\mu$g/mL, specifically 1$\mu$g/mL, 2$\mu$g/mL, 3$\mu$g/mL, 4$\mu$g/mL or 5$\mu$g/mL.

In some embodiments, the kit may further comprise a reagent for detecting the corresponding detectable label (the substrate of the detectable label described above). For example, when the detectable label is an enzyme, the kit may also comprise a colorimetric substrate for the corresponding enzyme, such as o-phenylenediamine (OPD), tetramethylbenzidine (TMB), ABTS or luminol compound for horseradish peroxidase, or p-nitrophenyl phosphate (p-NPP) or AMPPD for alkaline phosphatase. For example, when the detectable label is a chemiluminescent reagent (e.g., an acridinium ester), the kit may also comprise a pre-excitation solution and/or an excitation solution for chemiluminescence.

In some embodiments, the kit comprises one or more of the following: a Tg calibrator, a detectably labeled substrate, a washing solution, a stop solution, and instructions for use.

The binding characteristics of the antibody of the present application are comparable to those of the main TgAbs autoantibodies present in the human population, and the antibody can compete with TgAbs in the sample for binding to Tg antigen. The TgAbs content in the sample can be determined by quantifying the enzyme-labeled antibody in the test group and the control group (without sample). Based on the measured TgAbs content, the individual who provided the sample can be further diagnosed.

Therefore, the immunoassay kit of the present application can be used to detect Tg and/or TgAbs, in particular the presence or level of Tg and/or TgAbs in a sample.

In some embodiments, the sample is from a human, such as a human body fluid (e.g., blood, serum or plasma).

In some embodiments, the immunoassay kit has a positive diagnostic rate of not less than 80% for thyroiditis (e.g., Hashimoto's thyroiditis), and/or a positive diagnostic rate of not less than 60% for goiter (e.g., Graves' disease).

In another aspect, the present application provides a kit for competitive ELISA, comprising a Tg antigen and a Tg antibody, wherein,

for a patient clinically diagnosed with thyroiditis patient, the consistency rate of the diagnosis result using the kit with the clinical diagnosis result is not less than 80%, and/or,

for a patient clinically diagnosed with goiter, the consistency rate of the diagnosis result using the kit with the clinical diagnosis result is not less than 60%.

In some embodiments, the Tg antibody is selected from the antibody or antigen-binding fragment thereof described in any of the foregoing.

Detection method

Based on the antibody and/or kit of the present application, the present application further provides a method for detecting the presence or level of Tg and/or TgAbs in a sample, which comprises a step of using the antibody or antigen-binding fragment thereof or immunoassay kit as described in any of the foregoing.
In some embodiments, the method comprises the following steps:
allowing the sample to react with the first reagent and the second reagent, and quantitatively analyzing to obtain the presence or concentration of TgAbs in the sample. In some embodiments, the reaction and quantitative analysis in a chemiluminescent immunoassay analyzer.
In some embodiments, the method further comprises a step of calibrating with a Tg antigen calibrator. In some embodiments, the Tg antigen calibrator is a natural or recombinant Tg antigen.
In some embodiments, the limit of detection (LOD) of TgAbs in the sample is <10 IU/mL, preferably <5 IU/mL, and more preferably < 1 IU/mL.
In some embodiments, the detection range of TgAbs in the sample is 0.1 to 5000 IU/mL, such as less than 4500 IU/mL, less than 4000 IU/mL or less than 3500 IU/mL.
In some embodiments, the method is an ELISA immunoassay, such as a competitive ELISA immunoassay.
In some embodiments, the sample is from a human, such as a human body fluid (e.g., blood, serum or plasma).

Use

Based on the quantitative detection of TgAbs, thyroid autoimmune diseases can be diagnosed.
Specifically, the present application provides a use of the antibody or antigen-binding fragment thereof or the kit as described in any of the foregoing in the manufacture of a kit, wherein the kit is used for:

(1) detecting the presence or level of Tg and/or TgAbs in a sample;

(2) diagnosing a thyroid autoimmune disease (e.g., thyroiditis or goiter, preferably Hashimoto's thyroiditis or Graves' disease) or a thyroid cancer (e.g., differentiated thyroid cancer).

In another aspect, the present application provides a diagnostic method, comprising the steps of detecting a sample from a subject using the antibody or antigen-binding fragment thereof or the kit as described in any of the foregoing, or detecting a sample from a subject using the method described in any of the foregoing, and quantifying TgAbs in the sample, the method being used to diagnose a thyroid autoimmune disease (e.g., thyroiditis or goiter, preferably Hashimoto's thyroiditis or Graves' disease) or a thyroid cancer (e.g., differentiated thyroid cancer).
In some embodiments, the sample is from a human, such as a human body fluid (e.g., blood, serum or plasma).

Definition of terms

In the present application, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present application, the definitions and explanations of the relevant terms are provided below.
As used herein, the term "specific binding" refers to a non-random binding reaction between two molecules (i.e., a binding molecule and a target molecule), such as a reaction between an antibody and an antigen to which it is directed. The binding affinity between two molecules can be described by a $K_D$ value. The $K_D$ value refers to the dissociation constant obtained by the ratio of kd (dissociation rate of a specific binding molecule-target molecule interaction; also known as koff) to ka (association rate of a specific binding molecule-target molecule interaction; also known as kon), or refers to kd/ka expressed in molar concentration (M). The smaller the $K_D$ value, the tighter the two molecules bind and the higher the affinity. In certain embodiments, an antibody that specifically binds to an antigen (or an antibody that is specific to an antigen) refers to an antibody that binds to the antigen with a $K_D$ of less than about $10^{-5}$ M, such as less than about $10^{-6}$ M, $10^{-7}$ M, $10^{-8}$ M, $10^{-9}$ M or $10^{-10}$ M or less. The $K_D$ value can be determined by methods well known in the art, such as using surface plasmon resonance (SPR) in a BIACORE instrument.

As used herein, the term "immunological assay" refers to an assay that utilizes specific interaction/binding affinity between an antigen and an antibody, and is generally used to detect the presence or level of a specific antigen or antibody in a sample. Such immunological assay is well known to those skilled in the art, including but not limited to enzyme immunoassay (EIA), chemiluminescent immunoassay (CLIA), radioimmunoassay (RIA), fluorescent immunoassay (FIA), Western blotting, immunoturbidimetry, surface plasmon resonance, etc. "Enzyme-linked immunosorbent assay" (i.e., ELISA) comprises binding an antigen or antibody to the surface of a solid phase carrier, and cross-linking an antigen- or antibody-related substance with an enzyme to form an enzyme conjugate, which, on the one hand, maintains the immune characteristics of binding to the corresponding antigen or antibody, and on the other hand, has enzyme activity. When the enzyme conjugate binds to the corresponding antigen or antibody, an enzyme-labeled antigen-antibody complex is formed. When the enzyme on the complex encounters the corresponding substrate, it can catalyze the hydrolysis, oxidation or reduction of the product, thereby producing a colored substance. Based on the presence or absence of the colored substance and its concentration, it can be indirectly inferred whether the corresponding antigen or antibody exists in the sample being tested and its level, thereby achieving the purpose of qualitative and quantitative determination. Among them, competitive ELISA has a variety of modes for detecting antibodies. The sample and enzyme-labeled antibody can compete with the solid phase antigen for binding, or the sample and antigen are added to the solid phase antibody for competitive binding, and then the enzyme-labeled antibody is added after washing to react with the antigen bound to the solid phase. For a detailed description of immunological detection, see, for example, Fundamental Immunology, Ch. 7 Paul, W., ed., 2nd edition, Raven Press, N.Y. (1989).

As used herein, the terms "antibody" and "monoclonal antibody" are used interchangeably and refer to an immunoglobulin molecule that is usually composed of two pairs of polypeptide chains (each pair having a light chain (LC) and a heavy chain (HC)). Antibody light chains can be classified as $\kappa$ (kappa) and $\lambda$ (lambda) light chains. Heavy chains can be classified as $\mu$, $\delta$, $\gamma$, $\alpha$ or $\varepsilon$, and define the isotypes of antibody as IgM, IgD, IgG, IgA and IgE, respectively. Each heavy chain consists of a heavy chain variable region (VH) and a heavy chain constant region (CH). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin to host tissues or factors, including various cells of the immune system (e.g., effector cells) and the first component (C1q) of the classical complement system. The VH and VL regions can also be subdivided into regions of high variability, called complementarity determining regions (CDRs), interspersed with more conservative regions called framework regions (FRs). Each $V_H$ and $V_L$ consists of three CDRs and four FRs arranged from the amino terminus to the carboxyl terminus in the following order: FR1, CDR1, FR2, CDR2, FR3, CDR3, FR4. The variable regions (VH and VL) of each heavy chain/light chain pair form antigen binding sites, respectively. The distribution of amino acids in each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to the amino acid residues in an antibody variable region that is responsible for antigen binding. There are three CDRs in each of the variable regions of the heavy and light chains, designated as CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, a person skilled in the art will readily identify the CDRs defined by each numbering system. Moreover, the correspondence between different numbering systems is well known to those skilled in the art (for example, see, Lefranc et al., Dev. Comparat. Immunol. 27:55-77, 2003).

In the present application, the CDRs contained in the antibody or antigen-binding fragment thereof of the present application can be determined according to various numbering systems known in the art. In certain embodiments, the CDRs contained in the antibody or antigen-binding fragment thereof of the present application are determined by the Kabat numbering system.

As used herein, the term "framework region" or "FR" residues refers to those amino acid residues in an antibody variable region other than the CDR residues defined above.

The term "antibody" is not limited to any particular method for producing antibody. For example, it includes recombinant antibody, monoclonal antibody, and polyclonal antibody. The antibody can be an antibody of different isotypes, for example, an IgG (e.g., IgG1, IgG2, IgG3, or IgG4 subtype), an IgA1, an IgA2, an IgD, an IgE, or an IgM antibody.

As used herein, the term "antigen-binding fragment" of antibody refers to a polypeptide comprising a fragment of a full-length antibody that retains the ability to specifically bind to the same antigen to which the full-length antibody binds and/or competes with the full-length antibody for specific binding to the antigen, which is also referred to as an "antigen-binding portion." See generally, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab, Fab', F(ab')$_2$, Fd, Fv, complementarity determining region (CDR) fragments, scFv, diabodies, single domain antibodies, chimeric antibodies, linear antibodies, and polypeptides that contain at least a portion of antibody sufficient to confer specific antigen binding ability on the polypeptide. Engineered antibody variants are reviewed by Holliger et al., 2005; Nat Biotechnol, 23: 1126-1136.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')$_2$ fragment" refers to an antibody fragment comprising two Fab fragments connected by a disulfide bridge on the hinge regions; the term "Fab' fragment" refers to a fragment obtained after reducing the disulfide bond connecting the two heavy chain fragments in the F(ab')$_2$ fragment, which consists of a complete light chain and a heavy chain Fd fragment (consisting of VH and CH1 domains).

As used herein, the term "Fv" refers to an antibody fragment consisting of the VL and VH domains of a single arm of antibody. The Fv fragment is generally considered to be the smallest antibody fragment that can form a complete antigen binding site. It is generally believed that the six CDRs confer antigen binding specificity to the antibody. However, even a single variable region (e.g., an Fd fragment, which contains only three CDRs specific for an antigen) can recognize and bind to the antigen, although its affinity may be lower than that of the complete binding site.

As used herein, the term "scFv" refers to a single polypeptide chain comprising VL and VH domains, wherein the VL and VH are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH$_2$-VL-linker-VH-COOH or NH$_2$-VH-linker-VL-COOH. Suitable prior art linkers consist of repeated GGGGS amino acid sequences or variants thereof. For example, a linker having the amino acid sequence (GGGGS)$_4$ may be used, but variants thereof may also be used (Holliger et al. (1993), Proc. Natl. Acad. Sci. USA 90: 6444-6448). Other linkers that can be used in the present application are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between the VH and VL of the scFv.

As used herein, the term "diabody" means that its VH and VL domains are expressed on a single polypeptide chain, but using a linker that is too short to allow pairing between the two domains of the same chain, thereby forcing the domains to pair with the complementary domains of another chain and create two antigen binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the meaning commonly understood by those skilled in the art, which refers to an antibody fragment composed of a single monomer variable antibody domain (e.g., a single heavy chain variable region) that retains the ability to specifically bind to the same antigen to which the full-length antibody binds. Single-domain antibody is also called nanobody.

Each of the above antibody fragments retains the ability to specifically bind to the same antigen to which the full-length antibody binds, and/or competes with the full-length antibody for specific binding to the antigen.

Antigen-binding fragment of antibody (e.g., the above antibody fragment) can be obtained from a given antibody (e.g., the antibody provided by the present application) using conventional techniques known to those skilled in the art (e.g., recombinant DNA technology or enzymatic or chemical cleavage methods), and the antibody antigen-binding fragment can be screened for specificity in the same manner as for the intact antibody.

In this article, unless the context clearly indicates otherwise, when referring to the term "antibody", it includes not only an intact antibody, but also an antigen-binding fragment of the antibody.

As used herein, the term "chimeric antibody" refers to an antibody in which a portion of its light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but in any case, it still retains the activity to bind to the target antigen (U.S.P 4,816,567 to Cabilly et al.; Morrison et al., Proc. Natl. Acad. Sci. USA, 81:6851 6855 (1984)). For example, the term "chimeric antibody" may include such an antibody (e.g., a human-mouse chimeric antibody) in which the heavy chain and light chain variable regions of the antibody are derived from a first antibody (e.g., a murine antibody), and the heavy chain and light chain variable regions of the antibody are derived from a second antibody (e.g., a human antibody).

As used herein, the term "humanized antibody" refers to a genetically engineered non-human antibody whose amino acid sequence is modified to increase homology with the amino acid sequence of a human antibody. Generally speaking, all or part of the CDR regions of a humanized antibody are derived from a non-human antibody (donor antibody), and all or part of the non-CDR regions (e.g., variable region FR and/or constant region) are derived from a human immunoglobulin (recipient antibody). Humanized antibodies generally retain the expected properties of the donor antibody, including, but not limited to, antigen specificity, affinity, reactivity, etc. The donor antibody may be a mouse, rat, rabbit, or non-human primate (e.g., cynomolgus monkey) antibody having the expected properties (e.g., antigen specificity, affinity, reactivity, etc.).

The chimeric antibody or humanized antibody of the present application can be prepared according to the sequence of the mouse monoclonal antibody prepared above. DNA encoding the heavy and light chains can be obtained from the target mouse hybridoma and engineered to contain non-mouse (e.g., human) immunoglobulin sequences using standard molecular biology techniques.

To prepare chimeric antibodies, methods known in the art can be used to connect mouse immunoglobulin variable regions to human immunoglobulin constant region. For example, the DNA encoding VH can be operably connected to another DNA molecule encoding the heavy chain constant region to obtain a full-length heavy chain gene. The sequence of the human heavy chain constant region gene is known in the art (see, for example, Kabat, E.A. et al. (1991) Sequences of Proteins of Immunological Interest, Fifth Edition, U.S. Department of Health and Human Services, NIH Publication No.91-3242), and DNA fragments containing these regions can be obtained by standard PCR amplification. The heavy chain constant region can be IgG1, IgG2, IgG3, IgG4, IgA, IgE, IgM or IgD constant region, but IgG1 or IgG4 constant region is generally preferred. For example, the DNA encoding VL can be operably connected to another DNA molecule encoding the light chain constant region CL to obtain a full-length light chain gene (as well as a Fab light chain gene). The sequence of human light chain constant region gene is known in the art (see, for example, Kabat, E.A. et al. (1991), Sequences of Proteins of Immunological Interest, Fifth Edition, U.S. Department of Health and Human Services, NIH Publication No. 91-3242), and DNA fragments containing these regions can be obtained by standard PCR amplification. The light chain constant region can be a κ or λ constant region, but a κ constant region is generally preferred.

To prepare humanized antibodies, mouse CDR regions can be inserted into human framework sequences using methods known in the art (see Winter's U.S. Patent No. 5,225,539; Queen et al.'s U.S. Patent No.os.5,530,101; 5,585,089; 5,693,762 and 6,180,370; and Lo, Benny, K.C., editor, in Antibody Engineering: Methods and Protocols, volume 248, Humana Press, New Jersey, 2004).

As used herein, the term "vector" refers to a nucleic acid vehicle into which a polynucleotide can be inserted. When a vector can express a protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmids; phagemids; cosmids; artificial chromosomes, such as yeast artificial chromosome (YAC), bacterial artificial chromosome (BAC) or P1-derived artificial chromosome (PAC); bacteriophages, such as λ phage or M13 phage, as well as animal viruses, etc. Animal viruses that can be used as vectors include, but are not limited to, retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including, but not limited to, promoter sequence, transcription initiation sequence, enhancer sequence, selection element, and reporter gene. In addition, the vector may also contain a replication origin.

As used herein, the term "host cell" refers to a cell that can be used to introduce a vector, including, but not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell, or human cell.

As used herein, the term "identity" is used to refer to the matching of sequences between two polypeptides or between two nucleic acids. When a position in both sequences being compared is occupied by the same base or amino acid monomer subunit (e.g., a position in each of the two DNA molecules is occupied by adenine, or a position in each of the two polypeptides is occupied by lysine), then the molecules are identical at that position. The "percent identity" between two sequences is a function of the number of matching positions shared by the two sequences divided by the number of positions being compared × 100. For example, if 6 out of 10 positions in two sequences match, then the two sequences have an identity of 60%. For example, the DNA sequences CTGACT and CAGGTT share 50% identity (3 out of a total of 6 positions match). Typically, two sequences are compared when they are aligned to produce maximum identity. Such an alignment can be achieved by using, for example, the method of Needleman et al. (1970) J. Mol. Biol. 48: 443-453, which can be conveniently performed by a computer program such as the Align program (DNAstar, Inc.). The percent identity between two amino acid sequences can also be determined using the algorithm of E. Meyers and W. Miller (Comput. Appl Biosci., 4:11-17 (1988)), which has been incorporated into the ALIGN program (version 2.0), using a PAM120 weight residue table, a gap length penalty of 12, and a gap penalty of 4. In addition, the percent identity between two amino acid sequences can be determined using the algorithm of Needleman and Wunsch (J Mol Biol. 48:444-453 (1970)), which has been incorporated into the GAP program of the GCG software package (available at www.gcg.com), using a Blossum 62 matrix or a PAM250 matrix, a gap weight of 16, 14, 12, 10, 8, 6, or 4, and a length weight of 1, 2, 3, 4, 5, or 6.

As used herein, the term "conservative substitution" refers to an amino acid substitution that does not adversely affect or change the expected properties of the protein/polypeptide comprising the amino acid sequence. For example, a conservative substitution can be introduced by standard techniques known in the art such as site-directed mutagenesis and PCR-mediated mutagenesis. Conservative amino acid substitutions include substitutions of amino acid residues with amino acid residues having similar side chains, such as substitutions with residues physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference.

In the present application, the terms "polypeptide" and "protein" have the same meaning and are used interchangeably. And in the present application, amino acids are generally represented by single-letter and three-letter abbreviations known in the art. For example, alanine can be represented by A or Ala.

As used herein, the term "subject" includes, but is not limited to, various animals, particularly mammals, such as human.

In this article, unless otherwise stated, "%" refers to mass percentage.

## Brief Description of the Drawings

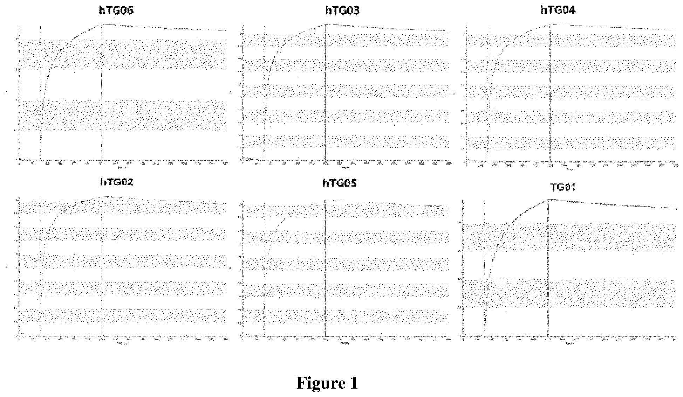

Figure 1 shows the binding curves of TG01, hTG02-hTG06 monoclonal antibodies.

## Embodiments of the Present Application

The embodiments of the present application will be described in detail below in conjunction with the examples, but those skilled in the art will understand that the following examples are only used to illustrate the present application and should not be regarded as limiting the scope of the present application. If the specific conditions were not specified in the examples, they were carried out according to conventional conditions or conditions recommended by the manufacturer. The reagents or instruments used without indicating the manufacturer were all conventional products that could be purchased commercially.

Example 1: Preparation of human thyroglobulin-specific monoclonal antibodies

Mice and rats were immunized with thyroglobulin from human thyroid (HyTest Cat# 8TG52, SDS-PAGE analysis purity > 90%). Six antibodies, TG01 to TG06, which were highly sensitive to anti-thyroglobulin autoantibody, were obtained through hybridoma screening (see Example 2 for the sensitivity test of the antibodies to anti-thyroglobulin autoantibody), in which TG01 was a mouse antibody and TG02 to TG06 were rat antibodies. These six antibodies were sequenced, and the results were shown in Tables 1 and 2.

Table 1: Sequencing results of antibody heavy chains

| Antibody name | | Heavy chain | | |
| --- | --- | --- | --- | --- |
| | | CDR1 | CDR2 | CDR3 |
| TG01 | Nucleotide sequence | aactatggaatgaac | tggataaacacctacactgga gagccaacatatgctgatgact tcaaggga | agaagagagacagctcggg ctcttgactcc |
| | Amino acid sequence | NYGMN | WINTYTGEPTYADD FKG | RRETARALDS |

(continued)

| Antibody name | | | Heavy chain | | |
| --- | --- | --- | --- | --- | --- |
| | | | CDR1 | CDR2 | CDR3 |
| TG02 | | Nucleotide sequence | gactatgacatggcc | tccattagtttagtcgtggtcgcacttattatcgagactccgtgaagggc | cagggacgatacagctcctttatcgatgcc |
| | | Amino acid sequence | DYDMA | SISFSRGRTYYRDSVKG | QGRYSSFIDA |
| TG03 | | Nucleotide sequence | cagtatgacatggcc | tccattagtccttttggtcctagaacttattatcgagactccgtgaagggc | cagggacggtactcctcctttatggatgcc |
| | | Amino acid sequence | QYDMA | SISPFGPRTYYRDSVKG | QGRYSSFMDA |
| TG04 | | Nucleotide sequence | caccatgacatggcc | tccattagttttagttatggtcgcacttattatcgagactccgtgaaggtc | cagggacgatacaactcctttatggatgcc |
| | | Amino acid sequence | HHDMA | SISFSYGRTYYRDSVKV | QGRYNSFMDA |
| TG05 | | Nucleotide sequence | aactatgacctggcc | tccattagtcgtagtcgtggtgacacttactatcgagactccgtaaagggc | caagattactatagcagcttccttgattac |
| | | Amino acid sequence | NYDLA | SISRSRGDTYYRDSVKG | QDYYSSFLDY |
| TG06 | | Nucleotide sequence | aattatggcatggcc | tccattacttatgatggtactagaacttactctcgagactccgtgaagggc | caccccgggtcctttgattac |
| | | Amino acid sequence | NYGMA | SITYDGTRTYSRDSVKG | HPGSFDY |

Table 2: Sequencing results of antibody light chains

| Antibody name | | | Light chain | | |
| --- | --- | --- | --- | --- | --- |
| | | | CDR1 | CDR2 | CDR3 |
| TG01 | | Nucleotide sequence | tgcagatctagtcagagccttgtacacagtaatggagacacctattta | tacaaagtttccaaccgattt | tgctctcaaagtacacatgttccattcacg |
| | | Amino acid sequence | CRSSQSLVHSNGDTYL | YKVSNRF | CSQSTHVPFT |

(continued)

| Antibody name | | Light chain | | |
| --- | --- | --- | --- | --- |
| | | CDR1 | CDR2 | CDR3 |
| TG02 | Nucleotide sequence | cggtcaagtcagagccttgtacaca gtgatggaaacacctacttgcat | cggggtttccaacagatt ttct | ttacaaagtacacattttcctcc gacg |
| | Amino acid sequence | RSSQSLVHSDGNTYLH | RVSNRFS | LQSTHFPPT |
| TG03 | Nucleotide sequence | cggtcaagtcagagccttgtacaca gtgatggaaacacctacttacat | cggggtttccaccagatt ttct | ttacaaagtacacattttcctcc gacg |
| | Amino acid sequence | RSSQSLVHSDGNTYLH | RVSTRFS | LQSTHFPPT |
| TG04 | Nucleotide sequence | cggtcaagtcagagccttgttcacag tgatggaaatacccacttgcat | cggggtgtccaacagat tctct | ttacaaagtacacattttcctcc gacg |
| | Amino acid sequence | RSSQSLVHSDGNTHLH | RVSNRFS | LQSTHFPPT |
| TG05 | Nucleotide sequence | cggtcaagtcagagcctggtacaca gtgatggaaaaacctacttacat | cggggtttccaacagatt ttct | ttgcaaagtacacattttcctc cgacg |
| | Amino acid sequence | RSSQSLVHSDGKTYLH | RVSNRFS | LQSTHFPPT |
| TG06 | Nucleotide sequence | aggtctagtcagagcctgctacatag taatggaaacacttatttggaa | aaggtttccaaccgagt ttct | ttccaagctacacatgatccg gtcacg |
| | Amino acid sequence | RSSQSLLHSNGNTYLE | KVSNRVS | FQATHDPVT |
| Note: The CDRs of the heavy chain variable regions and light chain variable regions mentioned above were defined by the Kabat numbering system. | | | | |

For the development of expression vectors, the variable domains of TG02, TG03, TG04, TG05 and TG06 rat antibodies and the constant domains of human immunoglobulin of heavy chain IgG1 isotype and light chain λ isotype were used. The expression vectors of the recombinant antibodies were obtained in a preparative amount in bacterial cells and purified. The mammalian cell line Expi293F was transfected with the expression vectors of the recombinant antibodies.

For example, the light chain and heavy chain gene sequences were transferred to the open reading frame (ORF) of the expression vector by homologous recombination or restriction enzyme digestion respectively, and finally the "promoter-antibody light chain gene-terminator" and "promoter-antibody heavy chain gene-terminator" structures were formed on the two expression vectors. Then, the two expression vectors (antibody light chain expression vector and heavy chain expression vector) were simultaneously transferred into the mammalian cell line Expi293F for expression by biological, physical or chemical methods. Finally, the two light chains and the two heavy chains were reassembled into a complete antibody in the cell and secreted into the culture supernatant. Alternatively, the expression vectors were modified so that one expression vector contained two open reading frames, and then the light chain and heavy chain gene sequences were inserted into the two open reading frames (ORF) of the expression vector by homologous recombination or restriction enzyme digestion, respectively, to form a "promoter-antibody light chain gene-terminator-vector sequence-promoter-antibody heavy chain gene-terminator" or "promoter-antibody heavy chain gene-terminator-vector sequence-promoter-antibody light chain gene-terminator" structure, and then the expression vector containing both antibody light chain and heavy chain gene sequences was transferred into the mammalian cell line Expi293F for expression by biological, physical and chemical methods. Finally, the two light chains and two heavy chains were reassembled into a complete antibody in the cell and secreted into the culture supernatant.

Antibodies were purified from conditioned medium by using protein A affinity chromatography. The resin was from GE health care Life Sciences (Piscataway, NJ) and purified according to the manufacturer's instructions. The above-mentioned recombinant chimeric antibodies hTG02 to hTG06 were obtained. The purified monoclonal antibodies were stored in 50% ammonium sulfate in the form of suspension at 4°C.

Deposit information:

TG01: The hybridoma cell line producing TG01 was deposited with VKPM on June 4, 2024, having Accession Number H-223.

hTG02: *Escherichia coli* Rosetta™(DE3)pLysS hTG02 VH was deposited with VKPM on June 26, 2024, having Accession Number B-14843, and the plasmid in the *E.coli* is used to produced hTG02 heavy chain. *Escherichia coli* Rosetta™(DE3)pLysS hTG02 VL was deposited with VKPM on June 26, 2024, having Accession Number B-14844, and the plasmid in the *E.coli* is used to produced hTG02 light chain.

hTG03: *Escherichia coli* Rosetta™(DE3)pLysS hTG03 VH was deposited with VKPM on June 26, 2024, having Accession Number B-14845, and the plasmid in the *E.coli* is used to produced hTG03 heavy chain. *Escherichia coli* Rosetta™(DE3)pLysS hTG03 VL was deposited with VKPM on June 26, 2024, having Accession Number B-14846, and the plasmid in the *E.coli* is used to produced hTG03 light chain.

hTG04: *Escherichia coli* Rosetta™(DE3)pLysS hTG04 VH was deposited with VKPM on June 26, 2024, having Accession Number B-14847,and the plasmid in the *E.coli* is used to producedhTG04 heavy chain. *Escherichia coli* Rosetta™(DE3)pLysS hTG04 VL was deposited with VKPM on June 26, 2024, having Accession Number B-14848, and the plasmid in the *E.coli* is used to produced hTG04 light chain.

hTG05: *Escherichia coli* Rosetta™(DE3)pLysS hTG05 VH was deposited with VKPM on June 26, 2024, having Accession Number B-14849, and the plasmid in the *E.coli* is used to produced hTG05 heavy chain. *Escherichia coli* Rosetta™(DE3)pLysS hTG05 VL was deposited with VKPM on June 26, 2024, having Accession Number B-14850, and the plasmid in the *E.coli* is used to produced hTG05 light chain.

hTG06: *Escherichia coli* Rosetta™(DE3)pLysS hTG06 was deposited with VKPM on June 26, 2024, having Accession Number B-14851, and the plasmid in the *E.coli* is used to produced hTG06.

Example 2: Detection of sensitivity of antibodies to anti-thyroglobulin autoantibody

The sensitivity of the developed monoclonal antibodies to thyroglobulin autoantibodies was determined by a competitive immunoassay method using an Eu-labeled thyroglobulin and a mixed serum sample containing high-titer anti-thyroglobulin autoantibody. First, the PBS solution for capturing anti-mouse or anti-rat IgG was incubated at 50 μL/well (2 μg/ml) in a 96-well plate with continuous shaking at room temperature for 30 minutes. The culture plate was washed with 10 mM Tris-HCl (pH 7.8) buffer supplemented with 0.15 M NaCl, 0.025% Tween-20 and 0.5 g/L $NaN_3$ (washing solution). The antibody solution to be tested was then added to the culture plate and incubated under the same conditions, followed by washing with washing solution. Next, native thyroglobulin labeled with a stable $Eu^{3+}$ chelate was pre-incubated with a buffer (50 mM Tris-HCl buffer, pH 7.8, 0.9% NaCl, 0.01% Tween-40, 0.5% BSA and 0.05% $NaN_3$) or a mixed serum with a final concentration of 30 ng/mL and a final level of 250 IU/mL of autoantibody for 15 minutes at room temperature, and then added to the culture plate. The plate was continuously shaken and incubated at room temperature for 30 minutes. After washing with washing solution, .1 ml of DELFIA® enhanced solution (Perkin Elmer, Finland) was added to each well and incubated with gentle shaking at room temperature for 10 minutes. The fluorescence of $Eu^{3+}$ was measured on a Victor 1420 multilabel counter (Wallac-Perkin Elmer, Finland). Fluorescence was expressed in counts per second (CPS). The selection of monoclonal antibodies with high sensitivity to anti-thyroglobulin autoantibody was based on the percentage of signal quenching, and calculation formula was as follows:

$$[\text{Tg-Eu} + \text{assay buffer}] \text{ cps} / [\text{Tg-Eu} + \text{master mix}] \text{ cps} * 100\%.$$

According to the results of the sensitivity analysis of anti-thyroglobulin autoantibody, TG01 and hTG02 to hTG06 all showed the highest sensitivity to anti-thyroglobulin autoantibody.

Example 3: Development of competitive chemiluminescent immunoassay

In order to perform continuous competitive chemiluminescent particle-based immunoassay, the detection monoclonal antibody was labeled with alkaline phosphatase (Catalog No. 03137031103, Roche Custom Biotech) and the capture antigen (recombinant thyroglobulin, HyTest Catalog No. 8RTG4) was labeled with biotin (Catalog No. PG82075, Thermo). The capture antigen at a concentration of 0.2 ug/mL was incubated with streptavidin-labeled particles (MyOne T1 Dynabeads Streptavidin PMP Hydrophobic Particles, 100 um, Cat# 35604D, Thermo), and the resulting antigen-labeled particles were prepared at a concentration of 0.25 mg/ml in a buffer solution containing 50 mM Tris-HCl, pH 7.4, 150 mM NaCl, 2% BSA, 0.05% γ-globulin (bovine), 0.2% Tween-20, 0.05% ProClin 300, and 0.09% $NaN_3$. The

detection monoclonal antibody was prepared at a concentration of 0.15 $\mu$g/ml in a buffer solution containing 50 mM MES, pH 6, 0.9% NaCl, 1% BSA, 0.05% $\lambda$-globulin (bovine), 0.05% Tween-20, 0.048% ProClin 300, 2 mM $MgCl_2$, 0.1 mM $ZnCl_2$. A pooled serum sample with a high level of anti-thyroglobulin autoantibody was serially diluted in a buffer solution containing 50 mM MES, pH 6, 0.9% NaCl, 1% BSA, 0.05% Tween-20, 0.048% ProClin 300, 2 mM $MgCl_2$, 0.1 mM $ZnCl_2$ for the generation of a calibration curve. Continuous competitive immunoassay was performed using an automatic analyzer Mindray CL-6000i according to the manufacturer's instructions (the first incubation was performed with the biotinylated Tg and sample, and the second incubation was performed with the labeled antibody). Signals were expressed in relative light units (RLU).

Determination of limit of detection (LOD)

Sixty replicates of zero analyte (50 mM MES, pH 6, 0.9% NaCl, 1% BSA, 0.05% Tween-20, 0.048% ProClin 300, 2 mM $MgCl_2$, 0.1 mM $ZnCl_2$) were analyzed in the corresponding competitive chemiluminescent immunoassay according to the recommendations (Testability Evaluation of Clinical Laboratory Measurement Procedures; Approved Guidelines -- Second Edition; EP17-A Vol. 24 No. 34) of the Clinical and Laboratory Standards Institute (CLSI). The general formula was:

$$LOB = 1,645 *$$

mean;

$$LOD = LOB + 1.645 * SD$$

Table 3: LOD values of 6 antibodies at end of CLIA

| Antigen | Antibody | LoD, IU/mL |
|---|---|---|
| Recombinant Tg antigen | hTG03 | 0.6 |
| | hTG06 | 1.2 |
| | TG01 | 0.4 |
| | hTG02 | 0.6 |
| | hTG04 | 0.6 |
| | hTG05 | 0.7 |

Example 4: Affinity detection of antibodies TG01, hTG02, hTG03, hTG04, hTG05, and hTG06

Affinity biolayer interferometry was performed according to the following protocol:
10 $\mu$g/mL of antibody buffer (50 mM Tris-HCl buffer, pH 7.8, 0.9% NaCl, 0.01% Tween-40, 0.5% BSA and 0.05% $NaN_3$) was coated on a sensor; the sensor was sealed; an antigen (recombinant thyroglobulin) at a concentration of 20 nM was applied; the complete experimental protocol comprised the following steps

(1) sensor hydration;
(2) antibody binding to the sensor;
(3) washing of the sensor;
(4) blocking of the sensor;
(5) association (for each concentration of the tested antibody);
(6) dissociation;
(7) regeneration of the sensor.

The results were shown in Figure 1 and Table 4.

Table 4: Affinity determination results of hTG06, hTG03, hTG04, hTG02, hTG05 and TG01

| Antibody | Ka(1/Ms) | Kd(1/s) | KD(M) |
|---|---|---|---|
| hTG06 | 1.13E+05 | 5.41E-05 | 4.77E-10 |
| hTG03 | 1.29E+05 | 7.59E-05 | 5.89E-10 |
| hTG04 | 1.35E+05 | 9.17E-07 | 6.79E-12 |
| hTG02 | 1.36E+05 | 9.55E-05 | 7.02E-10 |
| hTG05 | 1.33E+05 | 2.84E-05 | 2.13E-10 |
| TG01 | 7.15E+05 | 1.78E-05 | 2.49E-11 |

Example 5: Preparation of TgAbs detection kit and calibrator and detection of TgAbs

Step 1: Preparation of R1 reagent (containing Tg antigen)

The Tg antigen obtained by natural extraction or recombinant transformation was mixed well with superparamagnetic microparticles in a TBS buffer. After sufficient reaction, it was placed on a magnetic separator until the supernatant was clear. The supernatant was discarded, and the superparamagnetic microparticles coated with Tg antigen was retained. It was repeated 2-3 times with TBS buffer, the superparamagnetic microparticles coated with Tg antigen were dissolved with R1 reagent buffer; the R1 reagent was prepared, in which the content of superparamagnetic microparticles was 0.05%, the content of Tg antigen was 0.05%, and it was stored at 2-8°C for later use.

Step 2: Preparation of R2 reagent (containing detection antibody)

The specific Tg antibody was conjugated with alkaline phosphatase, then the conjugate was dissolved with R2 reagent buffer, in which the molar ratio of alkaline phosphatase to the antibody was 10:1, then the conjugate was dissolved with R2 diluent to prepare R2 reagent, in which the concentration of the conjugate was 5 $\mu$g/mL, and it was refrigerated at 2 to 8°C for later use. (R1 reagent and R2 reagent were combined to form a TgAbs kit)

Step 3: Preparation of TgAbs calibrators

TgAbs were diluted to reach concentrations at, such as 0 IU/mL, 10 IU/mL, 20 IU/mL, 50 IU/mL, 100 IU/mL, 300 IU/mL, 500 IU/mL, 1000 IU/mL, 2000 IU/mL, 3000 IU/mL, 4200 IU/mL, etc. respectively, thereby obtaining TgAbs calibrators.

Step 4: Measurement procedure of TgAbs kit

The TgAbs kit (containing R1 reagent and R2 reagent) was loaded into Mindray CL series fully automatic chemiluminescence immunoassay analyzer and tested according to the manual book of the analyzer;
The calibration test was performed using the matched TgAbs calibrators. Based on the calibration data, the system software used a weighted four-parameter logarithmic curve (4PLC) mathematical method to fit the luminescence signal to the concentration. The final result was given in the concentration form of IU/mL.
According to the above method, the TgAbs kits 1 to 5 (their antibody and antigen compositions were shown in Table 5 below) were obtained, and used to detect samples of patients with clinically confirmed thyroid inflammation and goiter.

Table 5: TgAbs kits

| TgAbs kit | Antigen | Detection antibody |
|---|---|---|
| Kit 1 | Tg recombinant antigen (Hytest, Cat. No.: 8RTG4) | hTG03 |
| Kit 2 | Tg recombinant antigen (Hytest, Cat. No.: 8RTG4) | hTG06 |
| Kit 3 | Tg recombinant antigen (Hytest, Cat. No.: 8RTG4) | TG01 |
| Kit 4 | Tg recombinant antigen (Hytest, Cat. No.: 8RTG4) | hTG02 |
| Kit 5 | Tg recombinant antigen (Hytest, Cat. No.: 8RTG4) | hTG04 |

(continued)

| TgAbs kit | Antigen | Detection antibody |
|---|---|---|
| Kit 6 | Tg recombinant antigen (Hytest, Cat. No.: 8RTG4) | hTG05 |

Detection items and results:

1. Consistency rate with clinical diagnosis

For samples of patients with thyroid inflammation (Hashimoto's thyroiditis) and goiter (Graves' disease) clearly confirmed with clinical diagnosis, the kits of the above examples were used for detection, and the results were judged according to the concentration value of TgAbs to determine its positive and negative performance, among which > 115 IU/mL was judged as positive (the number was recorded as n), and ≤ 115 IU/mL was judged as negative (the number was recorded as m). The positive rates of the kits of the examples were calculated, in which positive rate %=n/(n+m).

The results showed that the detection results of the current kits were highly consistent with the clinical diagnosis, and the positive rates could reach ≥80% in the samples of patients diagnosed with Hashimoto's thyroiditis, and the positive rates could reach ≥60% in the samples of patients diagnosed with goiter.

Table 6: Statistics of positive rates of samples of patients diagnosed with Hashimoto's thyroiditis

| Sample | Clinical diagnosis | Kit 1 | Kit 2 | Kit 3 | Kit 4 | Kit 5 | Kit 6 | Commercial Kit A* |
|---|---|---|---|---|---|---|---|---|
| 1 | Hashimoto's thyroiditis | 379.1 | 383.8 | 376 | 369.8 | 395.6 | 380.4 | 238.2 |
| 2 | Hashimoto's thyroiditis | 507.9 | 503.2 | 499.1 | 493.4 | 532.2 | 504.6 | 586.7 |
| 3 | Hashimoto's thyroiditis | 369.9 | 375.3 | 367.2 | 361 | 385.8 | 371.5 | 428.6 |
| 4 | Hashimoto's thyroiditis | 204.6 | 222.2 | 209.4 | 202.5 | 210.7 | 212.2 | 242.6 |
| 5 | Hashimoto's thyroiditis | 1230.9 | 1173 | 1189.7 | 1186.7 | 1298.7 | 1201.8 | 1305.6 |
| 6 | Hashimoto's thyroiditis | 108.2 | 109.9 | 155.5 | 148.4 | 150.9 | 157.8 | 100.2 |
| 7 | Hashimoto's thyroiditis | 64.2 | 92.1 | 75.3 | 67.8 | 61.8 | 76.8 | 54.2 |
| 8 | Hashimoto's thyroiditis | 170.7 | 175.8 | 173.3 | 166.3 | 166.9 | 187.2 | 278.9 |
| 9 | Hashimoto's thyroiditis | 38.9 | 55.9 | 54.6 | 47.1 | 42.5 | 72.1 | 56.2 |
| 10 | Hashimoto's thyroiditis | 3248 | 3241.6 | 3455.3 | 3324.6 | 3203.1 | 3167.9 | 3125.6 |
| 11 | Hashimoto's thyroiditis | 98.5 | 91.5 | 96.9 | 95.4 | 103 | 100.1 | 98.5 |
| 12 | Hashimoto's thyroiditis | 259.2 | 252.2 | 268.2 | 260.1 | 261.1 | 256.6 | 249.5 |
| 13 | Hashimoto's thyroiditis | 661.5 | 711.5 | 711.5 | 686.3 | 674.9 | 670.3 | 792.3 |
| 14 | Hashimoto's thyroiditis | 849.7 | 917.6 | 861.2 | 884.4 | 868.1 | 860.5 | 956.2 |
| 15 | Hashimoto's thyroiditis | 180 | 166.4 | 103.6 | 179.5 | 180.6 | 113.8 | 112.5 |
| 16 | Hashimoto's thyroiditis | 258.3 | 270.1 | 253.9 | 261.9 | 260.9 | 262.9 | 276.1 |
| 17 | Hashimoto's thyroiditis | 264.5 | 253 | 256.2 | 246.3 | 259.1 | 250.3 | 269.4 |
| 18 | Hashimoto's thyroiditis | 565.7 | 563.5 | 569.7 | 558.1 | 603.7 | 575.4 | 104.9 |
| 19 | Hashimoto's thyroiditis | 348 | 339.1 | 343.1 | 332.8 | 354.7 | 340.4 | 226.4 |
| 20 | Hashimoto's thyroiditis | 1008.3 | 1091.1 | 1024 | 1051.3 | 1030.9 | 1020.7 | 684.5 |
| Number of positive samples | | 16 | 16 | 16 | 16 | 17 | 17 | 16 |
| Positive rate | | 80% | 80% | 80% | 80% | 85% | 85% | 80% |
| Note: *Thyroglobulin antibody detection kit (electrochemiluminescence method), manufacturer: Roche, Germany. | | | | | | | | |

Table 7: Statistics of positive rates of samples of patients diagnosed with goiter

| Sample | Clinical diagnosis | Kit 1 | Kit 2 | Kit 3 | Kit 4 | Kit 5 | Kit 6 | Commercial Kit A* |
|---|---|---|---|---|---|---|---|---|
| 21 | Goiter | 350.90 | 346.80 | 363.20 | 340.50 | 348.50 | 355.40 | 308.50 |
| 22 | Goiter | 403.80 | 399.20 | 421.30 | 393.10 | 403.30 | 406.30 | 388.70 |
| 23 | Goiter | 40.40 | 39.20 | 21.80 | 31.70 | 26.50 | 57.20 | 25.60 |
| 24 | Goiter | 401.20 | 396.70 | 418.50 | 390.60 | 400.70 | 403.80 | 480.90 |
| 25 | Goiter | 311.50 | 307.80 | 319.90 | 301.30 | 307.60 | 317.60 | 331.80 |
| 26 | Goiter | 126.50 | 113.60 | 126.40 | 116.80 | 119.50 | 136.00 | 109.60 |
| 27 | Goiter | 27.50 | 26.40 | 17.10 | 18.40 | 12.60 | 44.30 | 25.30 |
| 28 | Goiter | 198.60 | 187.50 | 118.30 | 184.20 | 118.00 | 173.90 | 112.60 |
| 29 | Goiter | 721.50 | 713.90 | 770.60 | 709.10 | 732.80 | 711.50 | 680.90 |
| 30 | Goiter | 127.60 | 121.60 | 129.80 | 134.80 | 135.90 | 130.30 | 112.50 |
| 31 | Goiter | 96.80 | 96.80 | 99.60 | 91.80 | 93.30 | 95.10 | 96.50 |
| 32 | Goiter | 307.90 | 307.90 | 312.90 | 314.00 | 324.40 | 311.80 | 368.70 |
| 33 | Goiter | 287.20 | 287.20 | 292.10 | 292.30 | 301.70 | 290.60 | 269.80 |
| 34 | Goiter | 91.10 | 91.10 | 93.90 | 85.90 | 87.10 | 89.30 | 79.60 |
| 35 | Goiter | 114.20 | 114.20 | 107.80 | 183.30 | 178.00 | 174.50 | 104.60 |
| 36 | Goiter | 313.00 | 313.00 | 318.10 | 319.40 | 329.90 | 317.00 | 417.80 |
| 37 | Goiter | 102.90 | 102.90 | 108.20 | 110.60 | 114.30 | 108.40 | 101.60 |
| 38 | Goiter | 114.90 | 114.90 | 110.30 | 112.10 | 119.40 | 110.70 | 104.50 |
| 39 | Goiter | 536.60 | 536.60 | 544.00 | 527.90 | 533.50 | 570.40 | 517.60 |
| 40 | Goiter | 344.30 | 344.30 | 349.80 | 352.40 | 364.30 | 349.30 | 350.80 |
| Number of positive samples | | 13 | 13 | 12 | 13 | 14 | 15 | 14 |
| Positive rate | | 65% | 65% | 60% | 65% | 70% | 75% | 70% |
| Note: *Thyroglobulin antibody detection kit (electrochemiluminescence method), manufacturer: Roche, Germany. | | | | | | | | |

Example 6: Anti-interference effect of TgAbs detection kit (to be supplemented)

The anti-interference effect of the kit was evaluated according to the following scheme.

(1) Endogenous interference assessment (triglyceride, biotin, bilirubin, total protein):

Normal saline (0.9% NaCl solution) was used to prepare high-concentration triglyceride mother liquor, and samples with high and low concentration levels were divided into two groups on average. To one group of samples, triglyceride with the final concentration of 1500 mg/dL was added to make interference samples; to the other group, same volume of normal saline was added and used as control samples.

Normal saline (NaCl solution with concentration of 0.9%) was used to prepare high-concentration biotin mother liquor, and samples with high and low concentration levels were divided into two groups on average. To one group of samples, biotin with the final concentration of 3600 ng/mL was added to make interference samples; to the other, same volume of normal saline was added and used as control samples.

0.1 mol/L NaOH solution was used to prepare high concentration bilirubin mother liquor. Samples with high and low concentration levels were divided into two groups on average. To one group of samples, bilirubin mother liquor was added respectively to prepare interference samples containing 80 mg/dL bilirubin; to the other, same volume of 0.1 mol/L NaOH solution was added and used as control samples.

Samples with high and low concentration levels were divided into two groups on average. To one group, BSA (volume ignored) was added to prepare an interference sample of 15 g/dL total protein, and the other group was used as control samples.

The interference samples and the control samples were repeatedly tested twice. The mean value of test results of the interference samples was recorded as M, and the man value of test results of the control samples was recorded as T, and the relative deviation B1 was calculated according to the following formula, and the deviation should be within 10%.

$$B1=（M-T）/T×100\%$$

wherein,

B1-relative deviation;

M- the mean value of concentration of the interference sample;

T-the mean value of concentration of the control sample.

(2) ANA and RF interference: normal human serum samples were used as basic samples, and RF and ANA positive serum were used as RF and ANA positive samples. High concentration analyte samples were added to the basic sample and RF and ANA positive samples respectively, and the addition volume was controlled within 1/20 of the final volume to obtain the basic added sample and RF and ANA positive added sample. The mean value of test results of basic sample is recorded as X1, the mean value of test results of basic added sample is recorded as X2, the mean value of test results of autoantibody ANA and RF positive sample is recorded as Y1, and the mean value of test results of autoantibody ANA and RF positive added sample is recorded as Y2. The recovery deviation B2 of the measured concentration was calculated according to formula (2).

$$\text{Formula (2): } B2 = ((Y2-Y1)/(X2-X1)-1) \times 100\%;$$

The results show that the specific deviation of anti-endogenous interference and the specific deviation of anti-ANA and RF interference of the kit are all within acceptable standard range (10%), indicating that the kit of the invention has strong anti-endogenous interference ability and anti-ANA and RF interference ability.

The evaluation results are shown in Table 8 and Table 9.

Table 8 Evaluation results of anti-triglyceride, biotin, bilirubin and total protein interference of the kits

| Endogenous interference | Concentration | Groups | Result 1 (IU/m L) | Result 2 (IU/m L) | Mean value (IU/m L) | Deviation | Interference index | Conclusion |
|---|---|---|---|---|---|---|---|---|
| triglyceride | 1500 mg/dL | Negative blood control | 96.26 | 96.50 | 96.38 | 0% | ±10% | pass |
| | | Negative blood + tri-glyceride | 95.13 | 97.26 | 96.19 | | | |
| | | Positive blood control | 1561. 13 | 1611. 44 | 1586. 29 | 1% | ±10% | pass |
| | | Positive blood + tri-glyceride | 1585. 78 | 1603. 66 | 1594. 72 | | | |
| biotin | 3600 ng/mL | Negative blood control | 96.52 | 99.26 | 97.89 | -6% | ±10% | pass |
| | | Negative blood +biotin | 93.24 | 90.46 | 91.85 | | | |
| | | Positive blood control | 1576. 41 | 1559. 24 | 1567. 83 | 1% | ±10% | pass |
| | | Positive blood + biotin | 1604. 97 | 1558. 98 | 1581. 98 | | | |

(continued)

| Endogenous interference | Concentration | Groups | Result 1 (IU/mL) | Result 2 (IU/mL) | Mean value (IU/mL) | Deviation | Interference index | Conclusion |
|---|---|---|---|---|---|---|---|---|
| bilirubin | 80 mg/dL | Negative blood control | 80.33 | 78.93 | 79.63 | -3% | ±10% | pass |
| | | Negative blood +bilirubin | 78.72 | 76.33 | 77.53 | | | |
| | | Positive blood control | 1484.27 | 1530.55 | 1507.41 | 6% | ±10% | pass |
| | | Positive blood + bilirubin | 1569.43 | 1629.68 | 1599.56 | | | |
| Total protein | 15 g/dL | Negative blood control | 98.73 | 103.22 | 100.97 | -7% | ±10% | pass |
| | | Negative blood + total protein | 91.10 | 96.80 | 93.95 | | | |
| | | Positive blood control | 1583.81 | 1547.06 | 1565.44 | 1% | ±10% | pass |
| | | Positive blood + total protein | 1604.33 | 1563.15 | 1583.74 | | | |

Table 9 Evaluation results of anti-ANA and RF interference of the kits

| ANA/RF | Groups | Result 1 (IU/mL) | Result 2 (IU/mL) | Mean value (IU/mL) | Deviation | Interference index | Conclusion |
|---|---|---|---|---|---|---|---|
| Control | Basic sample | 10.73 | 11.96 | 11.35 | / | / | / |
| | Basic added sample 1 | 18.62 | 16.73 | 17.67 | / | / | / |
| | Basic added sample 2 | 22.89 | 21.31 | 22.10 | / | / | / |
| ANA | ANA positive sample | 12.69 | 12.39 | 12.54 | / | / | / |
| | ANA positive added sample 1 | 19.03 | 19.26 | 19.14 | 4% | ±10% | pass |
| | ANA positive added sample 2 | 25.83 | 22.73 | 24.28 | 4% | | |
| RF | RF positive sample | 8.74 | 11.09 | 9.91 | / | / | / |
| | RF positive added sample 1 | 15.48 | 16.72 | 16.10 | -2% | ±10% | pass |
| | RF positive added sample 2 | 19.14 | 20.36 | 19.75 | -9% | | |

The information of the sequences involved in the present application was described in the table below:

| SEQ ID NO: | Description | Sequence |
|---|---|---|
| 1 | H-CDR1 amino acid sequence of TG01 | NYGMN |
| 2 | H-CDR2 amino acid sequence of TG01 | WINTYTGEPTYADDFKG |
| 3 | H-CDR3 amino acid sequence of TG01 | RRETARALDS |
| 4 | L-CDR1 amino acid sequence of TG01 | CRSSQSLVHSNGDTYL |
| 5 | L-CDR2 amino acid sequence of TG01 | YKVSNRF |
| 6 | L-CDR3 amino acid sequence of TG01 | CSQSTHVPFT |
| 7 | H-CDR1 amino acid sequence of TG02/hTG02 | DYDMA |
| 8 | H-CDR2 amino acid sequence of TG02/hTG02 | SISFSRGRTYYRDSVKG |
| 9 | H-CDR3 amino acid sequence of TG02/hTG02 | QGRYSSFIDA |
| 10 | L-CDR1 amino acid sequence of TG02/hTG02 | RSSQSLVHSDGNTYLH |
| 11 | L-CDR2 amino acid sequence of TG02/hTG02 | RVSNRFS |
| 12 | L-CDR3 amino acid sequence of TG02/hTG02 | LQSTHFPPT |
| 13 | H-CDR1 amino acid sequence of TG03/hTG03 | QYDMA |
| 14 | H-CDR2 amino acid sequence of TG03/hTG03 | SISPFGPRTYYRDSVKG |
| 15 | H-CDR3 amino acid sequence of TG03/hTG03 | QGRYSSFMDA |
| 16 | L-CDR1 amino acid sequence of TG03/hTG03 | RSSQSLVHSDGNTYLH |
| 17 | L-CDR2 amino acid sequence of TG03/hTG03 | RVSTRFS |
| 18 | L-CDR3 amino acid sequence of TG03/hTG03 | LQSTHFPPT |
| 19 | H-CDR1 amino acid sequence of TG04/hTG04 | HHDMA |
| 20 | H-CDR2 amino acid sequence of TG04/hTG04 | SISFSYGRTYYRDSVKV |
| 21 | H-CDR3 amino acid sequence of TG04/hTG04 | QGRYNSFMDA |
| 22 | L-CDR1 amino acid sequence of TG04/hTG04 | RSSQSLVHSDGNTHLH |
| 23 | L-CDR2 amino acid sequence of TG04/hTG04 | RVSNRFS |

(continued)

| SEQ ID NO: | Description | Sequence |
|---|---|---|
| 24 | L-CDR3 amino acid sequence of TG04/hTG04 | LQSTHFPPT |
| 25 | H-CDR1 amino acid sequence of TG05/hTG05 | NYDLA |
| 26 | H-CDR2 amino acid sequence of TG05/hTG05 | SISRSRGDTYYRDSVKG |
| 27 | H-CDR3 amino acid sequence of TG05/hTG05 | QDYYSSFLDY |
| 28 | L-CDR1 amino acid sequence of TG05/hTG05 | RSSQSLVHSDGKTYLH |
| 29 | L-CDR2 amino acid sequence of TG05/hTG05 | RVSNRFS |
| 30 | L-CDR3 amino acid sequence of TG05/hTG05 | LQSTHFPPT |
| 31 | H-CDR1 amino acid sequence of TG06/hTG06 | NYGMA |
| 32 | H-CDR2 amino acid sequence of TG06/hTG06 | SITYDGTRTYSRDSVKG |
| 33 | H-CDR3 amino acid sequence of TG06/hTG06 | HPGSFDY |
| 34 | L-CDR1 amino acid sequence of TG06/hTG06 | RSSQSLLHSNGNTYLE |
| 35 | L-CDR2 amino acid sequence of TG06/hTG06 | KVSNRVS |
| 36 | L-CDR3 amino acid sequence of TG06/hTG06 | FQATHDPVT |
| 37 | H-CDR1 nucleotide sequence of TG01 | aactatggaatgaac |
| 38 | H-CDR2 nucleotide sequence of TG01 | tggataaacacctacactggagagccaacatatgctgatgacttcaaggga |
| 39 | H-CDR3 nucleotide sequence of TG01 | agaagagagacagctcgggctcttgactcc |
| 40 | L-CDR1 nucleotide sequence of TG01 | tgcagatctagtcagagccttgtacacagtaatggagacacctattta |
| 41 | L-CDR2 nucleotide sequence of TG01 | tacaaagtttccaaccgattt |
| 42 | L-CDR3 nucleotide sequence of TG01 | tgctctcaaagtacacatgttccattcacg |
| 43 | H-CDR1 nucleotide sequence of TG02/hTG02 | gactatgacatggcc |
| 44 | H-CDR2 nucleotide sequence of TG02/hTG02 | tccattagtttttagtcgtggtcgcacttattatcgagactccgtgaagggc |
| 45 | H-CDR3 nucleotide sequence of TG02/hTG02 | cagggacgatacagctcctttatcgatgcc |

(continued)

| SEQ ID NO: | Description | Sequence |
|---|---|---|
| 46 | L-CDR1 nucleotide sequence of TG02/hTG02 | cggtcaagtcagagccttgtacacagtgatggaaacacctacttgcat |
| 47 | L-CDR2 nucleotide sequence of TG02/hTG02 | cgggtttccaacagattttct |
| 48 | L-CDR3 nucleotide sequence of TG02/hTG02 | ttacaaagtacacattttcctccgacg |
| 49 | H-CDR1 nucleotide sequence of TG03/hTG03 | cagtatgacatggcc |
| 50 | H-CDR2 nucleotide sequence of TG03/hTG03 | tccattagtccttttggtcctagaacttattatcgagactccgtgaagggc |
| 51 | H-CDR3 nucleotide sequence of TG03/hTG03 | cagggacggtactcctcctttatggatgcc |
| 52 | L-CDR1 nucleotide sequence of TG03/hTG03 | cggtcaagtcagagccttgtacacagtgatggaaacacctacttacat |
| 53 | L-CDR2 nucleotide sequence of TG03/hTG03 | cgggtttccaccagattttct |
| 54 | L-CDR3 nucleotide sequence of TG03/hTG03 | ttacaaagtacacattttcctccgacg |
| 55 | H-CDR1 nucleotide sequence of TG04/hTG04 | caccatgacatggcc |
| 56 | H-CDR2 nucleotide sequence of TG04/hTG04 | tccattagttttagttatggtcgcacttattatcgagactccgtgaaggtc |
| 57 | H-CDR3 nucleotide sequence of TG04/hTG04 | cagggacgatacaactcctttatggatgcc |
| 58 | L-CDR1 nucleotide sequence of TG04/hTG04 | cggtcaagtcagagccttgttcacagtgatggaaatacccacttgcat |
| 59 | L-CDR2 nucleotide sequence of TG04/hTG04 | cgggtgtccaacagattctct |
| 60 | L-CDR3 nucleotide sequence of TG04/hTG04 | ttacaaagtacacattttcctccgacg |
| 61 | H-CDR1 nucleotide sequence of TG05/hTG05 | aactatgacctggcc |
| 62 | H-CDR2 nucleotide sequence of TG05/hTG05 | tccattagtcgtagtcgtggtgacacttactatcgagactccgtaaagggc |
| 63 | H-CDR3 nucleotide sequence of TG05/hTG05 | caagattactatagcagcttccttgattac |
| 64 | L-CDR1 nucleotide sequence of TG05/hTG05 | cggtcaagtcagagcctggtacacagtgatggaaaaacctacttacat |
| 65 | L-CDR2 nucleotide sequence of TG05/hTG05 | cgggtttccaacagattttct |
| 66 | L-CDR3 nucleotide sequence of TG05/hTG05 | ttgcaaagtacacattttcctccgacg |
| 67 | H-CDR1 nucleotide sequence of TG06/hTG06 | aattatggcatggcc |

(continued)

| SEQ ID NO: | Description | Sequence |
|---|---|---|
| 68 | H-CDR2 nucleotide sequence of TG06/hTG06 | tccattacttatgatggtactagaacttactctcgagactccgtgaagggc |
| 69 | H-CDR3 nucleotide sequence of TG06/hTG06 | cacccccgggtcctttgattac |
| 70 | L-CDR1 nucleotide sequence of TG06/hTG06 | aggtctagtcagagcctgctacatagtaatggaaacacttatttggaa |
| 71 | L-CDR2 nucleotide sequence of TG06/hTG06 | aaggtttccaaccgagtttct |
| 72 | L-CDR3 nucleotide sequence of TG06/hTG06 | ttccaagctacacatgatccggtcacg |

Although the specific modes of the present application have been described in detail, those skilled in the art will understand that various modifications and substitutions can be made to those details based on all the teachings disclosed, and these changes are within the scope of protection of the present application. The full scope of the present application is given by the appended claims and any equivalents thereof.

## Claims

1. An antibody or antigen-binding fragment thereof that specifically binds to Tg (thyroglobulin), which comprises:

   a heavy chain variable region comprising the following three CDRs: CDR-H1 having an amino acid sequence selected from SEQ ID NOs: 1, 7, 13, 19, 25, 31 and variants thereof, CDR-H2 having an amino acid sequence selected from SEQ ID NOs: 2, 8, 14, 20, 26, 32 and variants thereof, and/or CDR-H3 having an amino acid sequence selected from SEQ ID NOs: 3, 9, 15, 21, 27, 33 and variants thereof; and/or,
   a light chain variable region comprising the following three CDRs: CDR-L1 having an amino acid sequence selected from SEQ ID NOs: 4, 10, 16, 22, 28, 34 and variants thereof, CDR-L2 having an amino acid sequence selected from SEQ ID NOs: 5, 11, 17, 23, 29, 35 and variants thereof, and/or CDR-L3 having an amino acid sequence selected from SEQ ID NOs: 6, 12, 18, 24, 30, 36 and variants thereof;
   wherein each variant contains an amino acid mutation as compared to the amino acid sequence from which it is derived, and the amino acid mutation is a substitution, deletion or addition of one or more amino acids (for example, the substitution, deletion or addition of 1, 2, 3, 4 or 5 amino acids); preferably, the substitution is a conservative substitution; preferably, the variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived.

2. The antibody or antigen-binding fragment thereof according to claim 1, which comprises:

   (1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;
   (1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;
   (1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof; or

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof.

3. The antibody or antigen-binding fragment thereof according to claim 1 or 2, which further comprises a heavy chain constant region (CH) and a light chain constant region (CL);

preferably, the heavy chain constant region is a mouse heavy chain constant region, and the light chain constant region is a mouse light chain constant region;

preferably, the heavy chain constant region is a rat heavy chain constant region, and the light chain constant region is a rat light chain constant region;

preferably, the heavy chain constant region is a human heavy chain constant region, and the light chain constant region is a human light chain constant region;

preferably, the antibody or antigen-binding fragment thereof is an IgG, IgM, IgE, IgD or IgA antibody;

preferably, the heavy chain constant region is an IgG heavy chain constant region, such as an IgG1, IgG2, IgG3 or IgG4 heavy chain constant region;

preferably, the light chain constant region is a $\kappa$ or $\lambda$ light chain constant region (e.g., a human $\lambda$ light chain constant region).

4. The antibody or antigen-binding fragment thereof according to any one of claims 1 to 3, wherein the antigen-binding fragment is selected from scFv, Fab, Fab', (Fab')$_2$, Fd, Fv, CDR fragment, nanobody, disulfide bond-linked Fv (dsFv), diabody, bispecific antibody and multispecific antibody; and/or, the antibody is a mouse antibody, a chimeric antibody or a humanized antibody.

5. An antibody or antigen-binding fragment thereof that specifically binds to Tg, wherein the antibody or antigen-binding fragment thereof is:

produced based on a hybridoma cell line deposited with the All-Russian National Collection of Industrial Microorganisms (VKPM) having Accession Number H-223;

produced based on a plasmid in *Escherichia coli* Rosetta™(DE3)pLysS hTG02 VH deposited with VKPM having Accession Number B-14843, and a plasmid in *Escherichia coli* Rosetta™(DE3)pLysS hTG02 VL deposited with VKPM having Accession Number B-14844;

produced based on a plasmid in *Escherichia coli* Rosetta™(DE3)pLysS hTG03 VH deposited with VKPM having Accession Number B-14845, and a plasmid in *Escherichia coli* Rosetta™(DE3)pLysS hTG03 VL deposited with VKPM having Accession Number B-14846;

produced based on a plasmid in *Escherichia coli* Rosetta™(DE3)pLysS hTG04 VH deposited with VKPM having Accession Number B-14847, and a plasmid in *Escherichia coli* Rosetta™(DE3)pLysS hTG04 VL deposited with VKPM having Accession Number B-14848;

produced based on a plasmid in *Escherichia coli* Rosetta™(DE3)pLysS hTG05 VH deposited with VKPM having Accession Number B-14849, and a plasmid in *Escherichia coli* Rosetta™(DE3)pLysS hTG05 VL deposited with VKPM having Accession Number B-14850; or

produced based on a plasmid in *Escherichia coli* Rosetta™(DE3)pLysS hTG06 deposited with VKPM having Accession Number B-14851.

6. The antibody or antigen-binding fragment thereof according to any one of claims 1 to 5, which further has a detectable label;

fluorescein, chemiluminescence, enzymes, radioisotope, biotin, colloidal gold and magnetic particles; and preferably, the label is selected from the group consisting of fluorescein, acridinium esters, horseradish peroxidase, alkaline phosphatase, radioisotope, biotin, colloidal gold and magnetic particles.

7. The antibody or antigen-binding fragment thereof according to any one of claims 1 to 6, which competes with TgAbs (thyroglobulin antibodies) for binding to Tg.

8. An isolated nucleic acid molecule, which encodes the antibody or antigen-binding fragment thereof according to any one of claims 1 to 7.

9. An expression vector, which comprises the isolated nucleic acid molecule according to claim 8;
preferably, the vector is a plasmid, a virus, a phage, a bacterium or a viroid.

10. A host cell, which comprises the isolated nucleic acid molecule according to claim 8 or the expression vector according to claim 9;

preferably, the host cell is an eukaryotic cell, preferably a mammalian cell;
preferably, the host cell is a prokaryotic cell, preferably *Escherichia coli.*

11. An immunoassay kit, comprising:

a first reagent, which contains a Tg antigen, preferably, the Tg antigen is a natural or recombinant Tg antigen;
a second reagent, which contains at least one kind of antibody or antigen-binding fragment thereof that specifically binds to Tg, comprising:

a heavy chain variable region comprising the following three CDRs: CDR-H1 having an amino acid sequence selected from SEQ ID NOs: 1, 7, 13, 19, 25, 31 and a variant thereof, CDR-H2 having an amino acid sequence selected from SEQ ID NOs: 2, 8, 14, 20, 26, 32 and a variant thereof, and/or CDR-H3 having an amino acid sequence selected from SEQ ID NOs: 3, 9, 15, 21, 27, 33 and a variant thereof; and/or,
a light chain variable region comprising the following three CDRs: CDR-L1 having an amino acid sequence selected from SEQ ID NOs: 4, 10, 16, 22, 28, 34 and a variant thereof, CDR-L2 having an amino acid sequence selected from SEQ ID NOs: 5, 11, 17, 23, 29, 35 and a variant thereof, and/or CDR-L3 having an amino acid sequence selected from SEQ ID NOs: 6, 12, 18, 24, 30, 36 and a variant thereof.

12. The immunoassay kit according to claim 11, wherein the second reagent comprises:

(1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;
(1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;
(1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 hav

ing an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof; or

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof.

13. A kit for competitive ELISA, comprising a Tg antigen and a Tg antibody, wherein,

for a patient clinically diagnosed with thyroiditis, the consistency rate between the diagnosis result using the kit and the clinical diagnosis result is not less than 80%, and/or,

for a patient clinically diagnosed with goiter, the consistency rate between the diagnosis result using the kit and the clinical diagnosis result is not less than 60%.

14. A method for detecting the presence or level of Tg and/or TgAbs in a sample, which comprises a step of using the antibody or antigen-binding fragment thereof according to any one of claims 1 to 7 or the kit according to any one of claims 11 to 13 for detection.

15. Use of the antibody or antigen-binding fragment thereof according to any one of claims 1 to 7 in the manufacture of a kit or use of the kit according to any one of claims 11 to 13, wherein the kit is used for:

(1) detecting the presence or level of Tg and/or TgAbs in a sample; or
(2) diagnosing a thyroid autoimmune disease preferably thyroiditis or goiter, more preferably Hashimoto's thyroiditis or Graves' disease or a thyroid cancer preferably differentiated thyroid cancer.

**Figure 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5919632 A, Bergmann, A
- US 4816567 P, Cabilly
- US 5225539 A
- US 5530101 A, Queen
- US 5585089 A
- US 5693762 A
- US 6180370 B

**Non-patent literature cited in the description**

- Fundamental Immunology. Raven Press, 1989
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **KABAT et al.** Sequences of Proteins of Immunological Interest. Public Health Service, National Institutes of Health, 1991
- **LEFRANC et al.** *Dev. Comparat. Immunol.*, 2003, vol. 27, 55-77
- **HOLLIGER et al.** *Nat Biotechnol*, 2005, vol. 23, 1126-1136
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of Monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **MORRISON et al.** *Proc. Natl. Acad. Sci. USA*, 1984, vol. 81, 6851-6855
- **KABAT, E.A. et al.** Sequences of Proteins of Immunological Interest. U.S. Department of Health and Human Services, 1991
- Antibody Engineering: Methods and Protocols. Humana Press, 2004, vol. 248
- **NEEDLEMAN et al.** *J. Mol. Biol.*, 1970, vol. 48, 443-453
- **E. MEYERS** ; **W. MILLER**. *Comput. Appl Biosci.*, 1988, vol. 4, 11-17
- **NEEDLEMAN** ; **WUNSCH**. *J Mol Biol.*, 1970, vol. 48, 444-453
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991
- Testability Evaluation of Clinical Laboratory Measurement Procedures; Approved Guidelines. Clinical and Laboratory Standards Institute (CLSI), vol. 24